# EUROPÄISCHE PATENTSCHRIFT

(11) **EP 1 833 442 B1**
(45) Veröffentlichungstag und Bekanntmachung des Hinweises auf die Patenterteilung: **08.10.2008**
(21) Anmeldenummer: 06776591.7
(22) Anmeldetag: 03.08.2006
(51) Int. Cl.: A61F 9/013, A61B 17/32

(54) **VORRICHTUNG ZUM BESTÜCKEN EINES SCHNEIDKLINGENHALTERS EINES MIKROCHIRURGISCHEN SCHNEIDINSTRUMENTS MIT EINER SCHNEIDKLINGENEINHEIT**
DEVICE FOR FITTING A CUTTING BLADE HOLDER OF A MICROSURGICAL CUTTING INSTRUMENT WITH A CUTTING BLADE UNIT
DISPOSITIF POUR EQUIPER UN SUPPORT DE LAME DE COUPE D'UN INSTRUMENT DE COUPE MICROCHIRURGICAL AVEC UNE UNITE DE LAME DE COUPE

(30) Priorität: 12.08.2005 EP 05017649
(43) Veröffentlichungstag der Anmeldung: 19.09.2007
(73) Patentinhaber: WaveLight AG, 91058 Erlangen (DE)
(72) Erfinder: JEGLORZ, Tobias, 90425 Nürnberg (DE); DONITZKY, Christof, 90542 Eckental (DE)
(74) Vertreter: Katérle, Axel
(86) Internationale Anmeldenummer: PCT/EP2006/007699
(87) Internationale Veröffentlichungsnummer: WO 2007/019966

(56) Entgegenhaltungen:
- WO-A-00/57799
- US-A1- 2003 045 895
- US-A1- 2004 010 277

## Beschreibung

Die Erfindung betrifft eine Vorrichtung zum Bestücken eines Schneidklingenhalters eines mikrochirurgischen Schneidinstruments, insbesondere eines Schneidinstruments zur Verwendung bei refraktiven Augenbehandlungen, mit einer Schneidklingeneinheit.

Zur refraktiven Sehfehlerbehandlung des menschlichen Auges ist es bekannt, mittels eines als Mikrokeratom bezeichneten mikrochirurgischen Schneidinstruments ein Scheibchen (Flap) von der Hornhautoberfläche so zu trennen, dass es an einer Seite (Hinge) noch mit der Hornhaut verbunden ist. Durch Anheben und Wegklappen des Flaps wird das Hornhautinterface (Stroma) zur Hornhautneuformung zugänglich. Die Neuformung erfolgt typischerweise mittels eines Excimerlasers (z.B. 193nm), der die freigelegten Hornhautbereiche geeignet ablatiert. Nach Beendigung der Laserbehandlung wird der Flap zurückgeklappt.

Das Mikrokeratom weist üblicherweise eine auf den Augapfel aufzusetzende Saugringeinheit auf, an der ein mit einer Schneidklingeneinheit bestückter Schneidkopf (Schneidklingenhalter) gehalten ist. Die Saugringeinheit saugt sich durch Anlegen eines Vakuums am Augapfel (Limbus) fest und fixiert diesen so. Der Schneidkopf ist an der Saugringeinheit beweglich geführt und kann mittels einer elektromotorischen Antriebseinheit über die Hornhaut gefahren werden. Dabei schneidet eine Schneidklinge der Schneidklingeneinheit in die Hornhaut ein und trennt den Flap ab. Während des Vorwärtshubs des Schneidkopfs wird die Schneidklingeneinheit üblicherweise zu lateraler Oszillation angeregt.

Aus Hygienegründen ist es gängig, die Schneidklingeneinheit nach jeder Operation auszuwechseln. Das Einsetzen der einer frischen Schneidklingeneinheit in den Schneidkopf erfolgt bisher meist, indem die Schneidklingeneinheit unmittelbar mit der Hand oder unter Zuhilfenahme einer Pinzette in eine an dem Schneidkopf vorgesehene Aufnahmetasche für die Schneidklingeneinheit eingeführt wird. Nach Benutzung wird die Schneidklingeneinheit ebenfalls wieder mit bloßer Hand oder mit Hilfe einer Pinzette aus dem Schneidkopf herausgezogen und dann entsorgt. Es ist ohne weiteres nachvollziehbar, dass dabei die Gefahr von Verletzungen (die Schneidklingen sind äußerst scharf) und Kontaminationen ungeschützter Hände sehr groß ist. Durch Handverletzungen kann die Schneidklinge ihrerseits verunreinigt werden, was bei einer frischen Schneidklinge natürlich vermieden werden soll. Es besteht auch die Gefahr, dass die Schneidklinge, insbesondere ihre Schneidkante, durch unsachgemäße Handhabung beschädigt wird. Solche Beschädigungen der Schneidklinge können kaum merklich sein und dennoch das Scheidergebnis in höchstem Maß beeinträchtigen. Da es sich bei der Schneidklinge und dem Schneidkopf um Hochpräzisionsbauteile handelt, sind für das Einsetzen und Entfernen der Schneidklingeneinheit eine geschultes Auge und eine hohe Feinmotorik nötig, um nicht den fehlerfreien Einsatz des Mikrokeratoms zu gefährden.

WO 00/57799 A1 und US 2003/045895 A1 offenbaren beide jeweils eine Vorrichtung zum Bestücken eines augenchirurgischen Mikrokeratoms mit einer Schneidklingeneinheit, wobei die Vorrichtung einen magazinartigen Klingenhalter mit einer Aufnahmekammer für eine Schneidklingeneinheit umfasst. Mittels eines Schiebers kann die Schneidklingeneinheit aus der Aufnahmekammer in das Mikrokeratom eingeschoben werden.

US 2004/010277 A1 offenbart ebenfalls eine Vorrichtung zum Bestücken eines augenchirurgischen Mikrokeratoms mit einer Schneidklingeneinheit. Bei dieser Vorrichtung wird die Schneidklingeneinheit auf eine Führungsschiene aufgelegt, auf der zugleich das Mikrokeratom positioniert ist. Mittels eines an der Führungsschiene geführten Schiebers kann die Schneidklingeneinheit in das Mikrokeratom eingeschoben werden.

Aufgabe der Erfindung ist es, eine Vorrichtung zu schaffen, die ein einfaches, gefahrloses und beschädigungsfreies Einsetzen einer Schneidklingeneinheit in einen Schneidklingenhalter eines mikrochirurgischen Schneidinstruments, insbesondere zur Verwendung bei augenchirurgischen Behandlungen, gestattet.

Zur Lösung dieser Aufgabe ist erfindungsgemäß eine Vorrichtung mit den Merkmalen des Anspruchs 1 vorgesehen. Bevorzugte Weiterbildungen sind in den abhängigen Ansprüchen 2 bis 7 angegeben.

Die erfindungsgemäße Vorrichtung umfasst eine solche Vorrichtung erfindungsgemäß ein lösbar mit dem Schneidklingenhalter koppelbares Klingenmagazin, in dem mindestens eine Schneidklingeneinheit aufgenommen ist, sowie einen relativ zu dem KLingenmagazin schiebebeweglich gehaltenen oder halterbaren Schieber, mittels welchem die Schneidklingeneinheit aus dem Klingenmagazin in eine Klingenaufnahme des Schneidklingenhalters geschoben werden kann. Die erfindungsgemäße Lösung ermöglicht es, eine frische Schneidklingeneinheit ohne jede Körperberührung in den Schneidklingenhalter einzusetzen. Hierzu muss lediglich der Schieber betätigt werden, der die Schneidklingeneinheit aus dem Magazin herausdrückt und in die Klingenaufnahme des Schneidklingenhalters schiebt. Dadurch können die Sterilität und Unversehrtheit der Schneidklingeneinheit bewahrt werden. Auch sind keine Handverletzungen zu befürchten. Zudem sind die Kopplung des Magazins mit dem Schneidklingenhalter und die Betätigung des Schiebers für den Operateur oder sein Hilfspersonal keine komplizierten und mühevollen Tätigkeiten, insbesondere da das Magazin und der Schieber wegen ihrer Größe sehr viel leichter zu greifen und handzuhaben sind als die Schneidklingeneinheit alleine.

Um die Schneldklingeneinheit nicht nur in den Schneidklingenhalter einsetzen, sondern sie auch wieder herausziehen zu können, sind die Schneidklingeneinheit und der Schieber für eine schub- und zugkraftübertragende Kopplung miteinander ausgebildet. Hierzu kann ein distaler Koppelkopf des Schiebers (distal heißt hier fern von einem Betätigungsabschnitt, an dem Benutzer den Schieber anfasst und bedient) nach dem Schlüssel-Schloß-Prinzip mit einer geeigneten Koppelformation an der Schneidklingeneinheit im Eingriff bringbar sein.

Um das unsachgemäße Herausfallen der Schneidklingeneinheit aus dem Klingenmagazin zu vermeiden, sind dem Magazin vorzugsweise Sicherungsmittel zugeordnet, welche die Schneidklingeneinheit in dem Magazin sichern. Dabei können die Sicherungsmittel ein lösbares Sicherungselement umfassen, welches zum Eingriff in eine der Ankopplung der Schneidklingeneinheit an einen Klingenoszillationsantrieb dienende Vertiefung der Schneidklingeneinheit bestimmt und ausgebildet ist. Alternativ oder zusätzlich ist es möglich, dass die Sicherungsmittel Sicherungsformationen umfassen, welche für eine reibschlüssige Sicherung der Schneidklingeneinheit in dem Klingenmagazin sorgen.

Bei einer bevorzugten Ausführungsform ist der Schieber Teil einer gesondert von dem Magazin hergestellten, lösbar mit diesem koppelbaren Betätigungseinheit. Letztere kann beispielsweise ein mehrfach verwendbares Produkt sein, das nicht nach jedem Gebrauch auszutauschen ist. Das Magazin kann dagegen beispielsweise als Einmalprodukt vorgesehen sein, welches nach jedem Gebrauch komplett mit der Schneidklingeneinheit zu ersetzen ist. Es versteht sich, dass in einer alternativen Ausführungsform das Magazin und die Betätigungseinheit fest miteinander verbunden sein können, so dass dann stets das gesamte Bestückungsinstrument inklusive Magazin und Betätigungseinheit auszuwechseln ist. Bei zweiteiliger Ausführung kann die Betätigungseinheit beispielsweise eine den Schieber längsverschieblich aufnehmende Griffhülse aufweisen, welche im Bereich eines distalen Hülsenendes zur Kopplung mit dem Magazin ausgebildet ist.

Die Schneidklinge der Schneidklingeneinheit bildet an einem vorderen Klingenrand üblicherweise eine Schneidkante, wobei der gegenüberliegende, hintere Klingenrand mindestens Richtkante bilden kann, die der Ausrichtung der Schneidklingeneinheit in der Klingenaufnahme des Schneidklingenhalters dienen kann. Um die Schneidkante und bei Bedarf auch die Richtkante - letztere gewährleistet die erforderliche präzise Positionierung der Schneidklingeneinheit im Schneidklingenhalter - vor etwaiger Beschädigung beim herstellerseitigen Bestücken eines Magazins mit einer frischen Schneidklingeneinheit und auch beim Herausschieben der Schneidklingeneinheit aus dem Magazin zu schützen, ist das Magazin vorzugsweise derart gestaltet, dass bei ordnungsgemäß darin aufgenommener Schneidklingeneinheit die Schneidkante und gewünschtenfalls die Richtkante der Schneidklinge ringsum Freiraum haben. Es kann so gewährleistet werden, dass die Schneidkante und ggf. die Richtkante beim Einführen und Herausziehen der Schneidklingeneinheit in das bzw. aus dem Magazin nicht in Kontakt mit den Wänden des Magazins gelangen. Dies gewährleistet die Unversehrtheit dieser Kanten.

Das Magazin kann gemäß einer Ausführungsform zur Aufnahme einer einzigen Schneidklingeneinheit ausgestaltet sein, während es in einer abgewandelten Ausführungsform zur Aufnahme von zwei oder mehr Schneidklingeneinheiten ausgebildet ist. In letzterem Fall kann jede Schneidklingeneinheit in je einer eigenen Magazinkammer des Klingenmagazins untergebracht sein, wobei die erfindungsgemäße Bestückungsvorrichtung dann auch je einen Schieber in Zuordnung zu jeder Magazinkammer aufweisen kann. Es ist selbstverständlich nicht ausgeschlossen, dass mehrere Schneidklingeneinheiten in einer gemeinsamen Magazinkammer aufnehmbar sind.

Schutz wird im Rahmen der Erfindung ferner für ein Klingenmagazin zur Verwendung in einer Bestückungsvorrichtung der weiter oben beschriebenen zweigeteilten Art mit baulich vom Magazin getrennter Betätigungseinheit angestrebt. Das Magazin ist hierbei mit mindestens einer Schneidklingeneinheit bestückt. Außerdem wird Schutz angestrebt für eine Schneidklingeneinheit, welche mit mindestens einer Koppelformation ausgeführt ist, die zur zug- und schubkraftübertragenden Kopplung der Schneidklingeneinheit mit einem distalen Koppelkopf eines Schiebers einer erfindungsgemäßen Bestückungsvorrichtung bestimmt und ausgebildet ist.

Die Erfindung wird nachfolgend anhand der beigefügten Zeichnungen weiter erläutert. Es stellen dar:
Figur 1 in Perspektive ein Ausführungsbeispiel einer erfindungsgemäßen Bestückungsvorrichtung im gebrauchsbereiten Zustand,
Figur 2 perspektivisch ein Magazin und einen Teil einer Betätigungseinheit der Bestückungsvorrichtung der Figur 1,
Figur 3 eine Perspektivansicht einer Stirnseite des Magazins, welche in Gebrauchsposition einem Schneidkopf eines Mikrokeratoms zugewandt ist,
Figur 4 schematisch den Eingriff einer Betätigungsstange der Betätigungseinheit mit einer Schneidklingeneinheit,
Figur 5 ein Sicherungselement zur Sicherung der Schneidklingeneinheit in dem Magazin,
Figur 6 perspektivisch ein Magazin, einen Teil einer Betätigungseinheit sowie eine Schneidklingeneinheit gemäß einem weiteren Ausführungsbeispiel der erfindungsgemäßen Bestückungsvorrichtung und
Figur 7 eine axiale Draufsicht auf ein schneidkopfseitiges Ende der Bestückungsvorrichtung der Fig. 6.

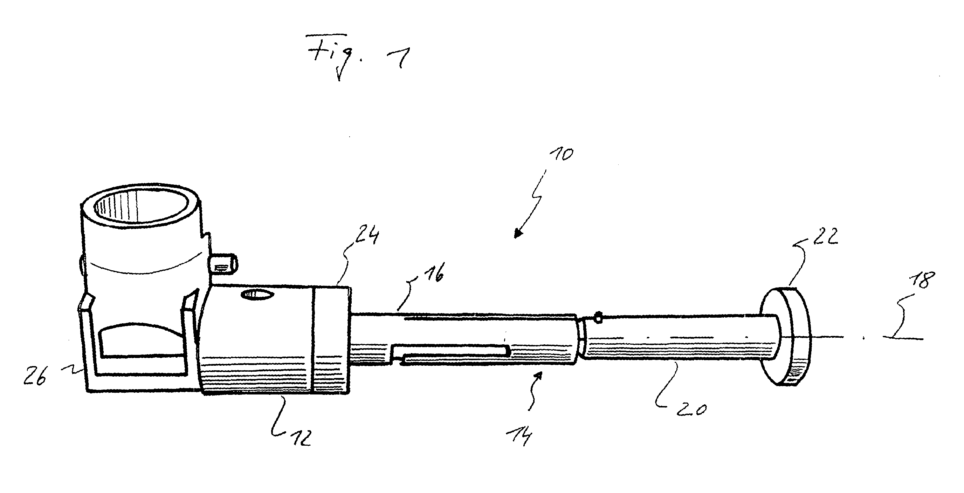

Die Bestückungsvorrichtung der Figur 1 ist allgemein mit 10 bezeichnet. Sie umfasst ein Magazin 12 sowie eine Betätigungseinheit 14 mit einer in einer Griffhülse 16 entlang einer Hülsenlängsachse 18 längsverschiebbar aufgenommenen Betätigungsstange 20. Die Betätigungsstange 20 bildet einen Schieber im Sinne der Erfindung und weist an ihrem proximalen Ende einen die Betätigung erleichternden Griffflansch 22 auf. Die Griffhülse 16 weist an ihrem distalen Ende einen Koppelabschnitt 24 auf, welcher der Ankopplung der Betätigungseinheit 14 an eine erste Axialseite des Magazins 12 dient. Im Beispielfall der Fig. 1 ist der Koppelabschnitt 24 als radial abstehender Koppelflansch 24 ausgeführt.

Das Magazin 12 ist auf seiner gegenüberliegenden zweiten (distalen) Axialseite zur lösbaren Kopplung mit einem Schneidklingenhalter (Schneidkopf) 26 eines Mikrokeratoms für augenchirurgische Behandlungen ausgebildet. Der Schneidkopf 26 weist eine in den Zeichnungen nicht näher dargestellte Aufnahmetasche für eine Schneidklingeneinheit auf, mittels welcher ein Flap von der Oberfläche der Augenhornhaut abgetrennt werden kann. Die Klingenaufnahmetasche des Schneidkopfs 26 ist zur Seite des Magazins 12 hin offen, so dass eine neue, unbenutzte Schneidklingeneinheit mit Hilfe der Betätigungseinheit 14 aus dem Magazin 12 in die Klingenaufnahmetasche des Schneidkopfs geschoben und nach Gebrauch aus dieser wieder herausgezogen werden kann.

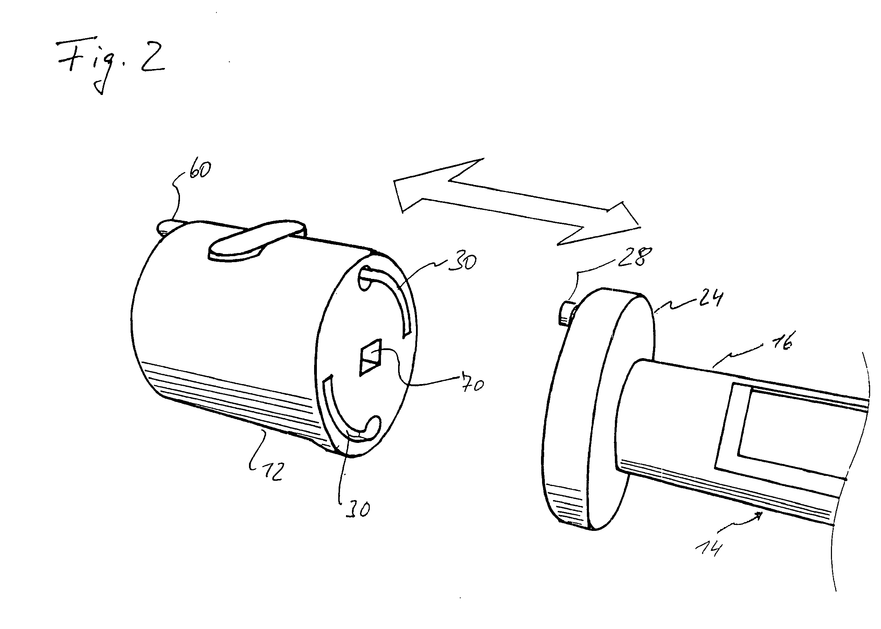

Es wird nun zusätzlich auf Figur 2 verwiesen. Die Betätigungseinheit 14 und das Magazin 12 sind baulich voneinander getrennt hergestellt und sind über zusammenwirkende Koppelformationen an der ersten Axialseite des Magazins 12 und am Koppelabschnitt 24 lösbar miteinander verbindbar. Im dargestellten Beispielfall bilden diese Koppelformationen einen Drehverschluss, wobei an einer der Komponenten: Magazin 12 und Koppelabschnitt 24 (hier dem Koppelabschnitt 24) eine Anordnung von zwei oder mehr axial abstehenden Verschlussstiften 28 mit einem querschnittsvergrößerten Stiftkopf vorgesehen ist. Die andere Komponente (hier das Magazin 12) weist in Zuordnung zu jedem der Verschlussstifte 28 je einen kreisbogenförmig sich erstreckenden Schlitz 30 auf, der im Bereich eines Schlitzendes breit genug ausgeführt ist, um das axiale Einführen des zugehörigen Verschlussstifts 28 in den betreffenden Schlitz 30 zu gestatten. Bei Relativdrehung des Magazins 12 und der Griffhülse 16 gleiten dann die Verschlussstifte 28 in den Schlitzen 30, wobei die querschnittsvergrößerten Stiftköpfe die Schlitzränder axial hintergreifen. So wird für einen sicheren Zusammenhalt des Magazins 12 und der Griffhülse 16 gesorgt. Die Qualität der Verbindung kann dadurch verbessert werden, dass die von den Stiftköpfen hintergriffenen Schlitzränder mit schräg zu einer achsnormalen Ebene ansteigenden Erhebungen oder Rampenflächen ausgeführt werden, sodass der Drehverschluss nach Art eines Gewinde-Bajonettverschlusses wirkt, welcher bei Relativdrehung des Magazins 12 und der Griffhülse 16 in geeigneter Drehrichtung eine axiale Anpressung dieser beiden Komponenten bewirkt. Alternativ oder zusätzlich können die Verschlussstifte 28 am Ende ihres Wegs in den Schlitzen 30 durch geeignete Rastmittel gesichert werden, sodass ein erhöhter Kraftaufwand erforderlich ist, um die Verschlussstifte 28 wieder aus ihrer Rastposition herauszubewegen und die Verbindung des Magazins 12 mit der Griffhülse 16 zu lösen.

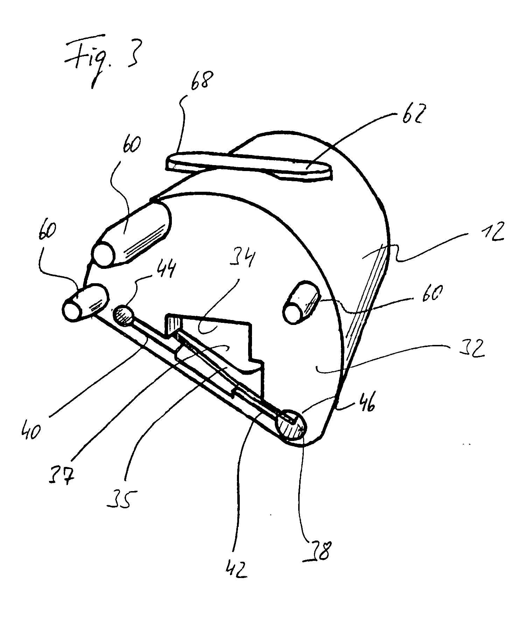

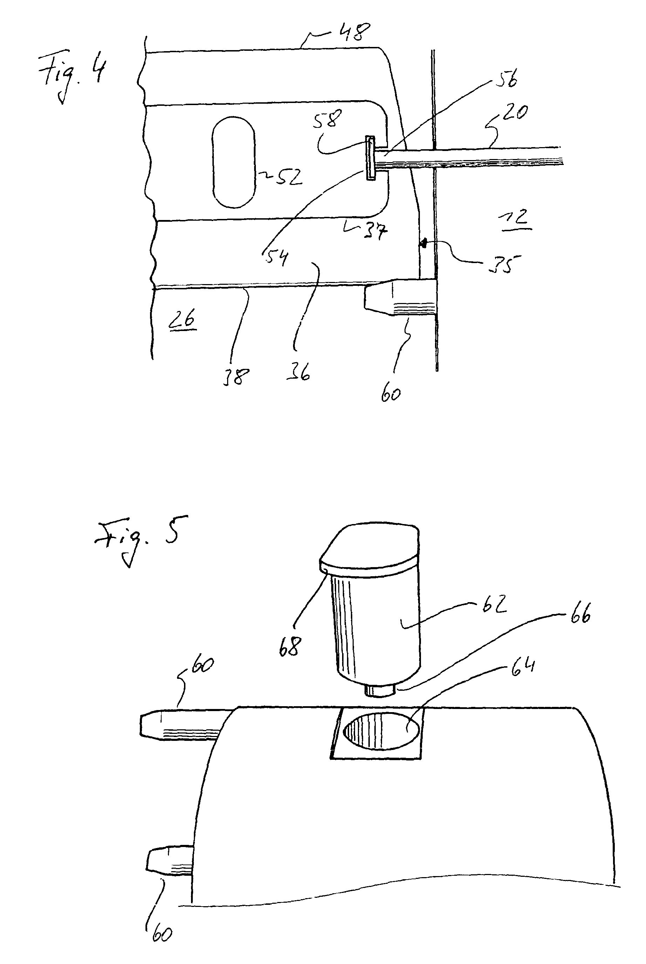

Es wird nun zusätzlich auf Figur 3 verwiesen. Wie in dieser Figur erkennbar, weist das Magazin 12 ein Magazingehäuse 32 auf, in dem eine Magazinkammer 34 ausgebildet ist. Die Magazinkammer 34 dient zur geschützten Aufnahme und Halterung einer Schneidklingeneinheit 35. Die Schneidklingeneinheit 35 weist eine Schneidklinge 36 sowie einen beispielsweise aus Kunststoff gefertigten und fest mit der Klinge 36 verbundenen Aufsatz 37 auf einer der Klingenflachseiten auf, siehe Fig. 4. An einem vorderen Klingenrand bildet die Schneidklinge 36 eine Schneidkante 38. Die Magazinkammer 34 ist in einem Mittelbereich querschnittsgrößer ausgebildet, um den Aufsatz 37 der Schneidklingeneinheit 35 aufnehmen zu können. Beidseits des querschnittsvergrößerten Mittelbereich weist die Magazinkammer 34 schlitzförmig verengte Abschnitte auf, wie in Figur 3 bei 40 und 42 angedeutet. An ihren Enden gehen die schlitzförmig verengten Abschnitte 40, 42 in querschnittserweiterte Kammerendbereiche 44, 46 über, in denen bei ordnungsgemäß in die Magazinkammer 34 eingesetzter Schneidklingeneinheit 35 die Schneidkante 38 sowie eine am hinteren Klingenrand gebildete Richtkante zu liegen kommen. Dabei haben die Schneidkante 38 und die in Figur 3 nicht näher erkennbare Richtkante der Schneidklinge 36 allseitigen Abstand von den Wänden der Magazinkammer 34, sodass keine Beschädigungsgefahr durch Kontakt mit den Kammerwänden besteht. Die querschnittserweiterten Kammerendbereiche 44, 46 können beispielsweise von Bohrungen gebildet sein, welche die schlitzartigen Abschnitte 40, 42 schneiden.

Zur weiteren Erläuterung der Schneidklinge 36 wird nun zusätzlich auf Figur 4 verwiesen. Die soeben erwähnte Richtkante der Schneidklinge 36 ist in dieser Figur mit 48 bezeichnet. Der Aufsatz 37 enthält eine zentrale, längliche Nut 52, die in an sich bekannter Weise für den Eingriff eines Exzenterstifts einer Antriebswelle einer nicht näher dargestellten elektromotorischen Antriebseinheit für die Schneidklinge 36 dient. Durch die Bewegung des Exzenterstifts in der Nut 52 wird die Schneidklinge 36 in seitliche Oszillation versetzt.

Seitlich ist in den Aufsatz 37 eine Koppelformation 54 eingeformt, die mit einem distalen Koppelkopf 56 der Betätigungsstange 20 in schub- und zugkraftübertragenden Eingriff bringbar ist. Der Eingriff zwischen der Koppelformation 54 der Schneidklingeneinheit 35 und dem Koppelkopf 56 ist form- und/oder kraftschlüssig. Im dargestellten Beispielfall der Figur 4 ist die Koppelformation 54 von einer hinterschnittenen T-Nut in dem Aufsatz 37 gebildet, in die der Koppelkopf 56 einführbar ist. Hierzu kann der Koppelkopf 56 eine radial abstehende Bundformation 58 tragen, welche derart ausgestaltet ist, dass in einer ersten Drehstellung der Betätigungsstange 20 die Bundformation 58 durch die Öffnung der Nut 54 hindurch in diese eingeführt und aus dieser herausbewegt werden kann, während in einer zweiten (beispielsweise um 90° gedrehten) Drehstellung der Betätigungsstange 20 die Bundformation 58 vor die Hinterschneidungsflächen der Nut 54 bewegt ist. Letztere Situation ist in Figur 4 dargestellt. In diesem Zustand ist die Betätigungsstange 20 an der Schneidklingeneinheit 35 verriegelt. Die hinterschnittene Nut 54 und die Bundformation 58 bilden einen Verriegelungsmechanismus nach dem Schlüssel-Schloß-Prinzip, wobei der Koppelkopf 56 den "Schlüssel" bildet und die Nut 54 das "Schloß" bildet. Es versteht sich, dass andere Ausführungsformen eines Verriegelungsmechanismus nach dem Schlüssel-Schloß-Prinzip vorstellbar sind. Es versteht sich ferner, dass die lösbare Kopplung der Betätigungsstange 20 mit der Schneidklingeneinheit 35 auch anderen mechanischen oder nicht-mechanischen Prinzipien folgen kann.

An seiner dem Schneidkopf 26 zugewandten Axialseite trägt das Magazin 12 eine Anordnung von Positionierformationen, mittels welcher das Magazin 12 am Schneidkopf 26 in einer das korrekte Einführen der Schneidklingeneinheit 35 in die Klingenaufnahmetasche gestattenden Lage positionierbar ist. Im dargestellten Beispielfall sind die Positionierformation des Magazins 12 von axial abstehenden Positionierstiften 60 gebildet, welche bei Aneinanderfügung des Magazins 12 und des Schneidkopfs 26 in zugeordnete Positionierlöcher (nicht näher dargestellt) des Schneidkopfs 26 eingreifen. Durch ein geeignetes Anordnungsbild der Positionierstifte 60 oder/und durch Variation der Dicke oder/und Länge der Positionierstifte kann gewährleistet werden, dass das Magazin 12 nur in einer einzigen, eindeutig definierten Relativorientierung ordnungsgemäß an den Schneidkopf 26 angesteckt werden kann. Die Kopplung zwischen dem Magazin 12 und dem Schneidkopf 26 kann eine lose Kopplung sein, sie kann aber auch durch die Wirkung geeigneter Haltemittel fixiert sein. Beispielsweise kann die Dicke der Positionierstifte 60 so auf den Durchmesser der an dem Schneidkopf 26 vorgesehenen Positionierlöcher abgestimmt sein, dass ein gewisser Reibschluss der Positionierstifte 60 in den Positionierlöchern auftritt, der für einen Zusammenhalt des Magazins 12 und des Schneidkopfs 26 sorgt. Alternative Möglichkeiten, um einen Halt des Magazins 12 an dem Schneidkopf 26 zu gewährleisten, umfassen beispielsweise die Vorsehung geeigneter Verrastungsmittel.

Es wird nun ferner auf Figur 5 verwiesen. Dort ist ein im wesentlichen stiftartiges Sicherungselement 62 zu erkennen, welches zur Sicherung der Schneidklingeneinheit 35 in dem Magazin 12 in eine von einer Außenumfangsfläche des Magazingehäuses 32 her in dieses eingearbeitete Bohrung 64 einsetzbar ist. Die Bohrung 64 öffnet sich in die Magazinkammer 34. An seinem bei ordnungsgemäßem Einsetzen in die Bohrung 64 vorlaufenden Ende weist das Sicherungselement 62 einen vorstehenden Sicherungszapfen 66 auf, welcher dazu bestimmt und ausgelegt ist, in die Nut 52 des Klingenaufsatzes 37 einzutauchen und hierdurch die Schneidklingeneinheit 35 gegen ungewolltes Herausfallen aus der Magazinkammer 34 zu sichern. Das Sicherungselement 62 ist zweckmäßigerweise durch geeignete Rastmittel in der Bohrung 66 verrastbar, so dass es nicht ungewollt aus der Bohrung 66 herausfällt. Es weist einen abstehenden Griffabschnitt 68 auf, der eine leichte Handhabung des Sicherungselements 62 gestattet.

In Figur 2 erkennt man noch, dass das Magazin 12 an seiner ersten Axialseite eine Durchtrittsöffnung 70 enthält, durch die die Betätigungsstange 20 mit ihrem Koppelkopf 56 hindurchtreten und in die Magazinkammer 34 gelangen kann.

Das Magazin 12 kann von einem Herstellerbetrieb als Einmalprodukt bereitgestellt werden, wobei es herstellerseitig bereits mit einer Schneidklingeneinheit 35 bestückt werden kann und diese dann in dem Magazin 12 beispielsweise mittels des Sicherungselements 62 oder auf andere Weise gesichert sein kann. Die Betätigungseinheit 14 kann als gesonderte Baueinheit hergestellt und angeboten werden, wobei sie als Einmalprodukt, aber auch als mehrfach verwendbares Produkt bestimmt sein kann.

Zum Zusammenbau der Bestückungsvorrichtung wird die Griffhülse 16 mit ihrem Koppelabschnitt 24 an dem Magazin 12 arretiert. Falls die Betätigungsstange 20 aus der Griffhülse 16 vollständig herausnehmbar ist, kann die Arretierung der Griffhülse 16 an dem Magazin 12 nach Wunsch bei herausgenommener Betätigungsstange 20 vorgenommen werden. Es ist selbstverständlich auch möglich, dass die Betätigungsstange 20 durch geeignete Anschlagmittel gegen vollständige Herausnahme aus der Griffhülse 16 gesichert ist.

Anschließend kann das Magazin 12 an den Schneidkopf 26 angesetzt werden und der Koppelkopf 56 der Betätigungsstange 20 mit der Koppelformation 54 der im Magazin 12 befindlichen Schneidklingeneinheit 35 in Eingriff gebracht werden. Nach Entfernung des Sicherungselements 62 - sofern vorhanden - kann sodann durch Druckausübung auf den Griffflansch 22 der Betätigungsstange 20 die Schneidklingeneinheit 35 aus der Magazinkammer 34 herausgedrückt und in die Klingenaufnahmetasche des Schneidkopfs 26 geschoben werden. Die schlitzartig verengten Abschnitte 40, 42 der Magazinkammer 34 bilden dabei eine Schiebeführung für die Schneidklinge 36. Die Schneidklinge 36 kann so definiert geführt in den Schneidkopf 26 hineinbewegt werden.

Nachdem die Schneidklingeneinheit 35 in den Schneidkopf 26 eingeführt wurde, wird die Bestätigungsstange 20 wieder von der Schneidklingeneinheit 35 gelöst, beispielsweise durch Drehen. Das Magazin 12 kann daraufhin zusammen mit der Betätigungseinheit 14 vom Schneidkopf 26 abgenommen werden.

Nach einer Behandlung mit der zuvor in den Schneidkopf eingesetzten Schneidklingeneinheit kann das Magazin 12 zusammen mit der Betätigungseinheit 14 wieder an den Schneidkopf 26 angesetzt werden und die Betätigungsstange 20 mit der im Schneidkopf 26 befindlichen Schneidklingeneinheit 35 gekoppelt werden. Durch Zurückziehen der Betätigungsstange 20 kann die Schneidklingeneinheit 35 zurück in das Magazin 12 überführt werden. In dem Magazin 12 kann die gebrauchte Schneidklingeneinheit 35 beispielsweise mittels des Sicherungselementes 62 wieder gesichert werden. Das Magazin 12 kann dann mit der darin befindlichen Schneidklingeneinheit 35 entsorgt werden.

Die verschiedenen Komponenten der Bestückungsvorrichtung 10 können aus Werkstoffen wie Kunststoff oder Metall hergestellt werden. Bei Einmalprodukten empfiehlt sich Kunststoff aufgrund der einfacheren Herstellbarkeit. Generell wird darauf zu achten sein, dass Werkstoffe verwendet werden, die sich gut sterilisieren lassen.

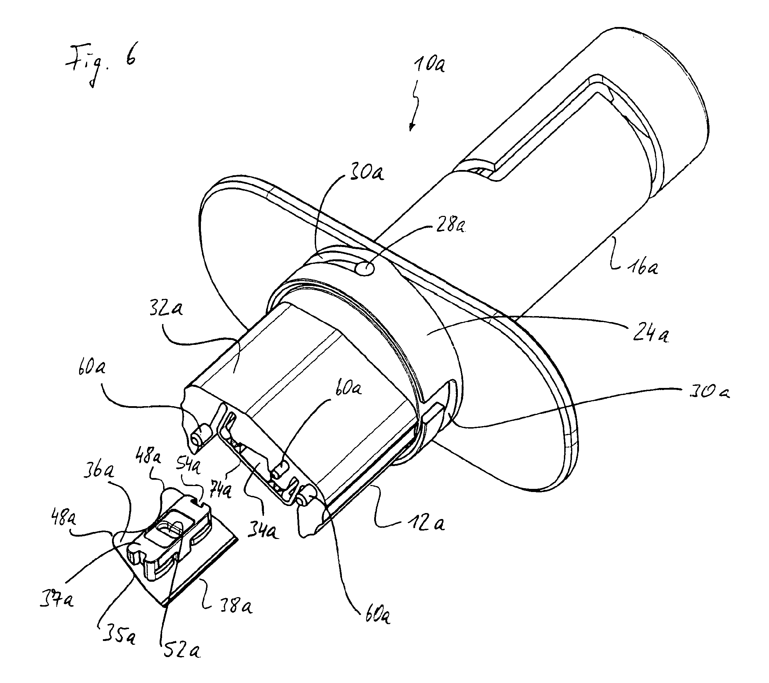

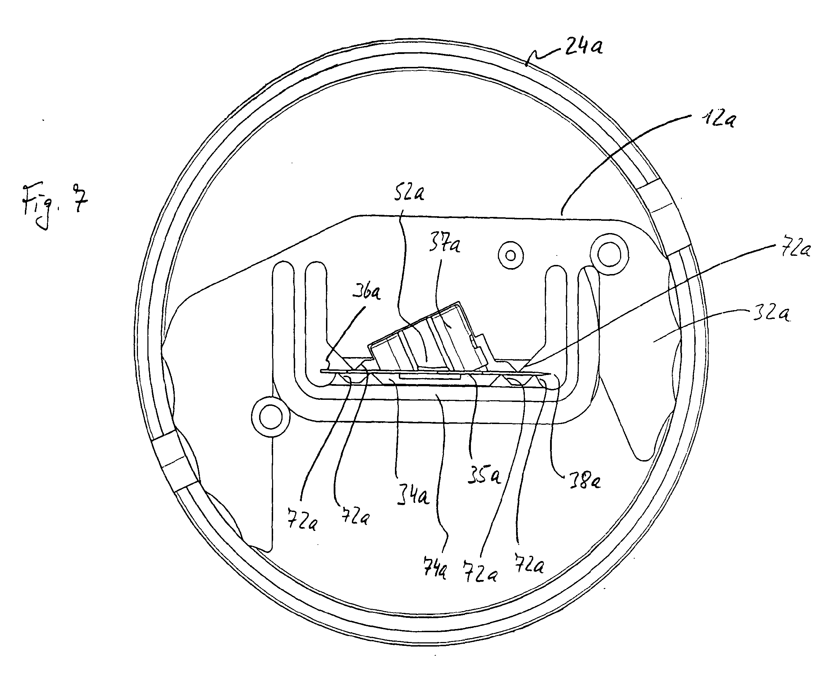

In den Figuren 6 und 7 sind gleiche oder gleichwirkende Komponenten mit gleichen Bezugszahlen wie zuvor bezeichnet, jedoch ergänzt durch einen Kleinbuchstaben. Bei dem in diesen Figuren gezeigten Ausführungsbeispiel ist die Schneidklingeneinheit 35a nicht durch einen gesondert an dem Magazin 12a anbringbaren Sicherungsstift in der Magazinkammer 34a sicherbar. Stattdessen sind in der Magazinkammer 34a Sicherungsformationen vorgesehen, welche die Schneidklingeneinheit 35a reibschlüssig in der Magazinkammer 34a halten und so verhindern, dass die Schneidklingeneinheit 35a ungewollt aus der Magazinkammer 34a herausfällt. Zur genauen Erläuterung dieser Sicherungsformationen wird insbesondere auf Figur 7 verwiesen. Dort erkennt man mehrere Sicherungsstege 72a, die auf beiden Seiten der Klingenebene der Schneidklinge 36a in der Magazinkammer 34a vorgesehen sind. Im gezeigten Beispielfall sind die Sicherungsstege 72a näherungsweise spitz zulaufend ausgeführt. Eine im Querschnitt rundliche Kontur der Sicherungsstege 72a ist selbstverständlich ebenso möglich. Die Sicherungsstege 72a sind so in der Magazinkammer 34a angeordnet, dass sie die Schneidklinge 36a und somit die Schneidklingeneinheit 35a zwischen sich halten, wenn die Schneidklingeneinheit 35a in die Magazinkammer 34a eingesetzt ist.

Man erkennt in Figur 7 ferner den Freiraum, der um die Schneidkante 38a und den hinteren Klingenrand der Schneidklinge 36a besteht, wenn die Schneidklingeneinheit 35a in die Magazinkammer 34a eingesetzt ist. Insgesamt besitzt die Magazinkammer 34a bei dem Ausführungsbeispiel der Figuren 6 und 7 einen näherungsweise U-förmigen Verlauf, wobei im Bereich des U-Stegs der zuvor schon im Zusammenhang mit dem Ausführungsbeispiel der Figuren 1 bis 5 erwähnte querschnittsverbreiterte Abschnitt zur Aufnahme des Aufsatzes 37a der Schneidklingeneinheit 35a vorgesehen ist. Auf der aufsatzfernen Flachseite der Schneidklinge 36a ist die Magazinkammer 34a durch eine nach Art eines U-Bügels verlaufende Begrenzungswand 74a begrenzt. Diese Begrenzungswand 74a ist auch gut in Figur 6 erkennbar.

Die Sicherungsstege 72a können beispielsweise einstückig mit dem Material des Magazingehäuses 33a ausgebildet sein. Das Magazingehäuse 32a seinerseits kann einteilig oder mehrteilig sein.

Die Sicherungsstege 72a können sich in Klingenquerrichtung, d.h. in Richtung senkrecht zur Zeichenebene der Figur 7, über eine Länge erstrecken, die wenigstens näherungsweise der Breite der Schneidklinge 36a entspricht. Für das Ziel der Sicherung der Schneidklingeneinheit 35a in der Magazinkammer 34a kann es auch genügen, die Sicherungsstege 72a als vergleichsweise kurze Stegsegmente auszuführen, die wesentlich kürzer als die Breite der Schneidklinge 36a sind. Anstelle von länglichen Sicherungsstegen oder -rippen ist es ferner vorstellbar, eine Mehrzahl noppenartiger Sicherungsvorsprünge auf beiden Seiten der Schneidklinge 36a in der Magazinkammer 34a vorzusehen. Solche Sicherungsnoppen können ebenfalls eine näherungsweise spitz zulaufende oder eine rundliche Kontur besitzen.

In Figur 6 erkennt man ferner, dass die Schneidklinge 36a an ihrem hinteren Klingenrand nicht eine einzige geradlinige Richtkante bildet, sondern mit zwei im Abstand voneinander vorgesehenen Anlagebereichen 48a ausgeführt ist, zwischen denen der hintere Klingenrand zurückversetzt ist. Die Anlageabschnitte 48a sind im gezeigten Beispielfall rundlich ausgeführt, sodass sie einen näherungsweise punktuellen Kontakt mit einer in dem Schneidkopf des Schneidinstruments vorgesehenen Widerlagerfläche herstellen können.

Als weiterer Unterschied zu dem Ausführungsbeispiel der Figuren 1 bis 5 sind bei dem Ausführungsbeispiel der Figuren 6 und 7 die Verriegelungsstifte 28a an dem Magazin 12a vorgesehen, während die Schlitze 30a in dem Koppelabschnitt 24a der Griffhülse 16a ausgebildet sind. Der Koppelabschnitt 24a ist hier als zylindrischer Abschnitt ausgebildet, welcher das Magazin 12a radial außen umgreift. Entsprechend sind die Schlitze 30a in der Zylinderwand des Koppelabschnitts 24a ausgebildet, während die Verriegelungsstifte 28a radial vom Außenumfang des Magazins 12a abstehen. Auch hier kann durch einen zur Umfangsrichtung schrägen Verlauf der Schlitze 30a eine Gewindewirkung erzielt werden, die für ein axiales Aneinanderdrücken der Griffhülse 16a und des Magazins 12a sorgen kann.

## Patentansprüche

1. Vorrichtung zum Bestücken eines Schneidklingenhalters (26) eines mikrochirurgischen Schneidinstruments, insbesondere eines Schneidinstruments zur Verwendung bei refraktiven Augenbehandlungen, mit einer Schneidklingeneinheit (35), umfassend
- ein lösbar mit dem Schneidklingenhalter koppelbares Klingenmagazin (12), in dem mindestens eine Schneidklingeneinheit (35) aufgenommen ist,
- einen relativ zu dem Magazin (12) schiebebeweglich gehaltenen oder halterbaren Schieber (20), mittels welchem die Schneidklingeneinheit (35) aus dem Klingenmagazin in eine Klingenaufnahme des Schneidklingenhalters (26) schiebbar ist,
**dadurch gekennzeichnet, dass** die Schneidklingeneinheit (35) und der Schieber (20) für eine schub- und zugkraftübertragende Kopplung miteinander ausgebildet sind.

2. Vorrichtung nach einem der vorhergehenden Ansprüche,
**dadurch gekennzeichnet, dass** dem Magazin (12) Sicherungsmittel zugeordnet sind, welche die Schneidklingeneinheit (35) in dem Magazin (12) sichern.

3. Vorrichtung nach Anspruch 2,
**dadurch gekennzeichnet, dass** die Sicherungsmittel ein lösbares Sicherungselement (62) umfassen, welches zum Eingriff in eine der Ankopplung der Schneidklingeneinheit (35) an einen Klingenoszillationsantrieb dienende Vertiefung (52) der Schneidklingeneinheit bestimmt und ausgebildet ist.

4. Vorrichtung nach Anspruche 2,
**dadurch gekennzeichnet, dass** die Sicherungsmittel Sicherungsformationen umfassen, welche für eine reibschlüssige Sicherung der Schneidklingeneinheit (35a) in dem Klingenmagazin (12a) sorgen.

5. Vorrichtung nach einem der vorhergehenden Ansprüche,
**dadurch gekennzeichnet, dass** der Schieber (20) Teil einer gesondert von dem Klingenmagazin (12) hergestellten, lösbar mit diesem koppelbaren Betätigungseinheit (14) ist.

6. Vorrichtung nach Anspruchs 5,
**dadurch gekennzeichnet, dass** die Betätigungseinheit (14) eine den Schieber (20) längsverschieblich aufnehmende Griffhülse (16) aufweist, welche im Bereich eines distalen Hülsenendes zur Kopplung mit dem Klingenmagazin (12) ausgebildet ist.

7. Vorrichtung nach einem der vorhergehenden Ansprüche,
**dadurch gekennzeichnet, dass** die Schneidklingeneinheit (35) eine Schneidklinge (36) mit einer Schneidkante (38) an einem Klingenrand und mindestens einer der Ausrichtung der Schneidklingeneinheit in der Klingenaufnahme des Schneidklingenhalters (26) dienenden Richtkante (48) an einem gegenüberliegenden Klingenrand aufweist und dass das Klingenmagazin (12) derart gestaltet ist, dass bei ordnungsgemäß darin aufgenommener Schneidklingeneinheit (35) die Schneidkante (38) und gewünschtenfalls die Richtkante (48) der Schneidklinge (36) ringsum Freiraum haben.

8. Klingenmagazin (12) zur Verwendung in einer Vorrichtung nach Anspruch 5 oder 6 oder nach Anspruch 7 soweit abhängig von Anspruch 5, wobei das Klingenmagazin mit mindestens einer Schneidklingeneinheit (35) bestückt ist.

9. Schneidklingeneinheit (35) zur Verwendung in einer Vorrichtung nach einem der Ansprüche 1 bis 7, wobei die Schneidklingeneinheit (35) mit mindestens einer Koppelformation (54) ausgeführt ist, welche zur zug- und schubkraftübertragenden Kopplung mit einem distalen Koppelkopf (56) eines Schiebers (20) der Vorrichtung bestimmt und ausgebildet ist.

## Claims

1. Device for fitting a cutting blade holder (26) of a microsurgical cutting instrument, in particular a cutting instrument for use in refractive eye treatments, with a cutting blade unit (35), said device comprising
- a blade cartridge (12) which is adapted to be detachably coupled to the cutting blade holder and in which at least one cutting blade unit (35) is received,
- a slide (20) which is or can be held slidably relative to the cartridge (12) and by means of which the cutting blade unit (35) can be pushed out of the blade cartridge into a blade receptacle of the cutting blade holder (26),
**characterised in that** the cutting blade unit (35) and the slide (20) are designed for thrust- and traction-transmitting coupling to one another.

2. Device according to one of the preceding claims,
**characterised in that** the cartridge (12) is assigned securing means securing the cutting blade unit (35) in the cartridge (12).

3. Device according to Claim 2,
**characterised in that** the securing means comprise a detachable securing element (62) which is intended and designed for engagement in a recess (52) of the cutting blade unit, which recess serves for coupling the cutting blade unit (35) to a blade oscillating drive.

4. Device according to Claim 2,
**characterised in that** the securing means comprise securing formations ensuring frictional securing of the cutting blade unit (35a) in the blade cartridge (12a).

5. Device according to one of the preceding claims,
**characterised in that** the slide (20) is part of an actuating unit (14) which is produced separately from and can be detachably coupled to the blade cartridge (12).

6. Device according to Claim 5,
**characterised in that** the actuating unit (14) comprises a gripping sleeve (16) which longitudinally displaceably receives the slide (20) and is designed in the region of a distal sleeve end for coupling to the blade cartridge (12).

7. Device according to one of the preceding claims,
**characterised in that** the cutting blade unit (35) has a cutting blade (36) with a cutting edge (38) at one blade edge and with at least one aligning edge (48) at an opposite blade edge, the aligning edge serving for aligning the cutting blade unit in the blade receptacle of the cutting blade holder (26), and **in that** the blade cartridge (12) is configured in such a way that, when the cutting blade unit (35) is correctly received therein, the cutting edge (38) and desirably the aligning edge (48) of the cutting blade (36) have a clearance all the way round.

8. Blade cartridge (12) for use in a device according to Claim 5 or 6 or according to Claim 7 as far as dependent on Claim 5, the blade cartridge being fitted with at least one cutting blade unit (35).

9. Cutting blade unit (35) for use in a device according to one of Claims 1 to 7, the cutting blade unit (35) being designed with at least one coupling formation (54) intended and designed for thrust- and traction-transmitting coupling to a distal coupling head (56) of a slide (20) of the device.

## Revendications

1. Dispositif pour équiper un support de lame de coupe (26) d'un instrument de coupe microchirurgical, en particulier d'un instrument de coupe pour une utilisation dans le cas de traitements réfractifs des yeux, avec une unité de lame de coupe (35), comprenant
- un chargeur de lames (12) pouvant être couplé de façon amovible avec le support de lames de coupe, dans lequel est reçue au moins une unité de lame de coupe (35),
- un coulisseau (20) maintenu ou pouvant être maintenu de façon mobile par coulissement par rapport au chargeur (12), coulisseau au moyen duquel l'unité de lame de coupe (35) peut coulisser à partir du chargeur de lame dans un logement de lame du support de lame de coupe (26),
**caractérisé en ce que** l'unité de lame de coupe (35) et le coulisseau (20) sont réalisés pour un couplage l'un avec l'autre transmettant la force de coulissement et la force de traction.

2. Dispositif selon l'une quelconque des revendications précédentes,
**caractérisé en ce que** des moyens de blocage sont associés au chargeur (12), lesquels bloquent l'unité de lame de coupe (35) dans le chargeur (12).

3. Dispositif selon la revendication 2,
**caractérisé en ce que** les moyens de blocage comprennent un élément de blocage (62) amovible qui est destiné à et conçu pour être engagé dans une cavité (52) de l'unité de lame de coupe, ladite cavité servant au couplage de l'unité de lame de coupe (35) à une commande d'oscillation de lame.

4. Dispositif selon la revendication 2,
**caractérisé en ce que** les moyens de blocage comprennent des formations de blocage qui garantissent un blocage par friction de l'unité de lame de coupe (35a) dans le chargeur de lames (12a).

5. Dispositif selon l'une quelconque des revendications précédentes,
**caractérisé en ce que** le coulisseau (20) fait partie d'une unité d'actionnement (14) réalisée séparément du chargeur de lames (12) et couplée de façon amovible avec celui-ci.

6. Dispositif selon la revendication 5,
**caractérisé en ce que** l'unité d'actionnement (14) présente un manchon poignée (16) recevant le coulisseau (20) avec un coulissement longitudinal, qui est réalisé dans la zone d'une extrémité de manchon distale pour le couplage avec le chargeur de lames (12).

7. Dispositif selon l'une quelconque des revendications précédentes,
**caractérisé en ce que** l'unité de lame de coupe (35) présente une lame de coupe (36) avec une arête de coupe (38) sur un bord de lame et au moins une arête d'alignement (48) servant à l'alignement de l'unité de lame de coupe dans le logement de lame du support de lame de coupe (26) sur un bord de lame opposé, et **en ce que** le chargeur de lames (12) est conçu de telle sorte que, lorsque l'unité de lame de coupe (35) est logée correctement à l'intérieur, l'arête de coupe (38) et éventuellement l'arête d'alignement (48) de la lame de coupe (36) ont tout autour un espace libre.

8. Chargeur de lames (12) pour une utilisation dans un dispositif selon la revendication 5 ou 6 ou selon la revendication 7, lorsque dépendante de la revendication 5, le chargeur de lames étant équipé d'au moins une unité de lame de coupe (35).

9. Unité de lame de coupe (35) pour une utilisation dans un dispositif selon l'une quelconque des revendications 1 à 7, l'unité de lame de coupe (35) étant réalisée avec au moins une formation de couplage (54) qui est destinée et conçue pour le couplage transmettant la force de traction et la force de poussée avec une tête de couplage (56) distale d'un coulisseau (20) du dispositif.
